# EUROPEAN PATENT APPLICATION

(11) **EP 3 747 411 A1**
(43) Date of publication of application: **09.12.2020**
(21) Application number: 19743599.3
(22) Date of filing: 29.01.2019
(51) Int. Cl.: A61F 11/00, A61B 1/227

(54) **EAR TREATMENT INSTRUMENT**

(30) Priority: 29.01.2018 JP 2018012311
(71) Applicant: KYOTO MEDICAL CONSUL, CO., LTD., Kyoto-shi, Kyoto 613-0911 (JP); Nobelpharma Co., Ltd., Chuo-ku Tokyo 103-0024 (JP); JMR Co., Ltd., Niigata-shi, Niigata 959-0511 (JP)
(72) Inventor: Shin-ichi Kanemaru, Ashiya-shi, Hyogo 659-0033 (JP)
(74) Representative: Schön, Christoph
(86) International application number: PCT/JP2019/003038
(87) International publication number: WO 2019/146806

(57) **Abstract**

The present invention provides an instrument for ear treatment that facilitates treatment or surgery in the ear by inserting a tool, such as a scalpel, into the instrument for ear treatment. An instrument 1 for ear treatment of the present invention includes an insertion portion 2 formed in a horn tubular shape and configured to be inserted into an external acoustic meatus, wherein a support portion 3 configured to support a tool to be inserted into the insertion portion 2 is provided on an inner circumferential surface 2a or at an open end on a distal end side of the insertion portion 2. The support portion 3 is preferably formed from a projection projecting from the inner circumferential surface 2a of the insertion portion 2 and the support portion 3 is preferably formed on a distal end side in a direction of an axis L1 of the insertion portion 2.

## Description

### Technical Field

The present invention relates to an instrument for ear treatment including an otoscope.

### Background Art

For treatment inside an ear by medical doctors, otoscopes are widely used allowing a straight view of a treatment area by preparing the external acoustic meatus linearly or allowing treatment or surgery by inserting a scalpel, an injection needle, and the like to project them from a distal end (e.g., PTL 1 below).

Such an otoscope in the past as described in PTL 1 is formed in a beheaded conical tube shape and has an insertion portion to be inserted into the external acoustic meatus. The insertion portion has an inner circumferential surface formed into a smooth circumferential surface gradually tapered from a base end toward a distal end.

Doctors practice treatment while inserting a tool, such as a scalpel, in the insertion portion of an otoscope and looking into the ear. For example, in a regenerative surgery of the tympanic membrane and the like, no wound has to be inflicted on a surrounding wall of the small ear hole with a diameter roughly from 3 mm to 9 mm when a scalpel or an injection is used for an incision in an edge of the tympanic membrane, filling with a medicine, and the like. Such treatment has to be precisely practiced by suppressing slight trembling of the hand to 0.3 mm or less and thus takes extremely high skills and a high degree of concentration.

### Citation List

### Patent Literature

PTL 1: JP 10-276978 A

### Summary of Invention

### Technical Problem

However, the otoscope in the past has an inner circumferential surface formed into a smooth circumferential surface and thus has no means of suppressing subtle trembling of the hand. The otoscope in the past also has a problem that, for example when part of a tool is placed on the inner circumferential surface as a fulcrum to move a distal end of the tool, the fulcrum sometimes slips. Moreover, such an operation has to be practiced highly precisely and thus the doctors practicing the operation are limited to skilled doctors.

The present invention has been made in view of the above circumstances and it is an object thereof to provide an instrument for ear treatment that facilitates treatment or surgery in the ear by inserting a tool, such as a scalpel.

An instrument for ear treatment according to the present invention is configured by improving part of a so-called otoscope. In the following description, the instrument herein may be referred to simply as an otoscope. Note that it is not intended to be limited to a so-called otoscope for ear observation.

In addition, a distal end side herein means a direction of the side to be inserted into the ear during use and a base end side herein means a direction located on an operator's side outside the ear during use.

### Solution to Problem

An instrument for ear treatment of the present invention includes an insertion portion formed in a horn tubular shape and configured to be inserted into an external acoustic meatus, wherein a support portion configured to support a tool to be inserted into the insertion portion is provided in arbitrary part of an inner circumferential surface or at an open end on a distal end side of the insertion portion.

This configuration allows suppression of tool shake with the support portion.

Preferably in the instrument for ear treatment of the present invention, the support portion is formed from a projection projecting from the inner circumferential surface of the insertion portion.

This configuration allows the tool to be supported with the support portion and the inner circumferential surface of the insertion portion, thereby satisfactorily suppressing the tool shake.

Preferably in the instrument for ear treatment of the present invention, the support portion is formed on a distal end side in a direction of an axis of the insertion portion.

This configuration allows a decrease in a distance between the position of the tool placed on the support portion and the distal end of the tool for surgery, thereby readily stabilizing operation of the tool. In this description, the distal end side in the direction of the axis of the insertion portion means an area up to half of the distal end side of the insertion portion, preferably an area up to one third of the distal end side, and more preferably an area up to one sixth of the distal end side.

Preferably in the instrument for ear treatment of the present invention, the insertion portion has a distal end portion as an inclined surrounding wall portion having a shape obtained by obliquely cutting a surrounding wall in a cylindrical shape relative to an axis of the surrounding wall, and the inclined surrounding wall portion has an inner circumferential surface formed with the support portion in arbitrary part.

This configuration allows surgery by supporting the tool with the support portion formed in the inclined surrounding wall portion while pressing a site closest to a surgical site against the inclined surrounding wall portion and also causes formation of no surrounding wall of the insertion portion in the other sites, thereby allowing a wider view of the entire affected area.

Preferably in the instrument for ear treatment of the present invention, the insertion portion has a distal end portion as an inclined surrounding wall portion having a shape obtained by obliquely cutting a surrounding wall in a cylindrical shape relative to an axis of the surrounding wall, and the support portion is formed on an inner circumferential surface on a shortest portion side of the inclined surrounding wall portion facing a longest portion of the distal end portion most projecting in a direction of an axis.

This configuration allows surgery by supporting the tool with the support portion while pressing a swelling and the like in an area other than the surgical site against the inclined surrounding wall portion to extend the vicinity of the affected surgical area.

Preferably in the instrument for ear treatment of the present invention, the inner circumferential surface is provided with a plurality of the support portions.

This configuration offers a plurality of options for the support portion to be used and thus facilitates setting the orientation of the insertion portion in accordance with the shape inside the ear.

Preferably in the instrument for ear treatment of the present invention, the support portion is formed in a columnar shape projecting from the inner circumferential surface toward the center.

This configuration allows a smooth change in the orientation of the distal end of the tool at the support portion along the circumferential surface of the support portion.

Preferably in the instrument for ear treatment of the present invention, the support portion is formed as a support in an O shape, a V shape, or a Y shape projecting from the inner circumferential surface toward the center.

This configuration allows stable placement of the tool at the center of the O shaped support portion or between branched areas of the V shaped or Y shaped support portion. At this point, when the support portion is formed in an O shape, a V shape, or a Y shape, the support portion is formed in an orientation appearing in an O shape, a V shape, or a Y shape when taken along the axis from the opening on the base end side.

Preferably in the instrument for ear treatment of the present invention, the support portion has a surface formed with a recess to support the tool.

This configuration allows placement of the tool in the recess and thus more facilitates suppression of slip of the tool on the support.

Preferably in the instrument for ear treatment of the present invention, the support portion has a height of 1 mm or more and 5 mm or less from the inner circumferential surface.

This configuration allows stable support of the tool with the support portion and also maximum prevention of interference with the field of view of the insertion portion.

Preferably in the instrument for ear treatment of the present invention, the insertion portion has an outer circumferential surface provided with a raised streak extending about the axis.

This configuration allows improvement in rigidity of the insertion portion.

Preferably in the instrument for ear treatment of the present invention, the insertion portion of the instrument for ear treatment is formed from a transparent material.

This configuration allows brightening inside the ear as much as possible and an indirect view of the area pressed against the insertion portion.

### Advantageous Effects of Invention

The instrument for ear treatment of the present invention exhibits an effect of facilitating surgery by supporting the tool to be inserted into the insertion portion.

### Brief Description of the Drawings

Fig. 1 is a perspective view illustrating an instrument for ear treatment according to an embodiment of the present invention.
Fig. 2 is a front view illustrating the instrument for ear treatment according to the above embodiment of the present invention.
Fig. 3 is a bottom view illustrating the instrument for ear treatment according to the above embodiment of the present invention.
Fig. 4 is a diagram of the instrument for ear treatment according to the above embodiment of the present invention taken from an opening on a base end side in a direction of an axis.

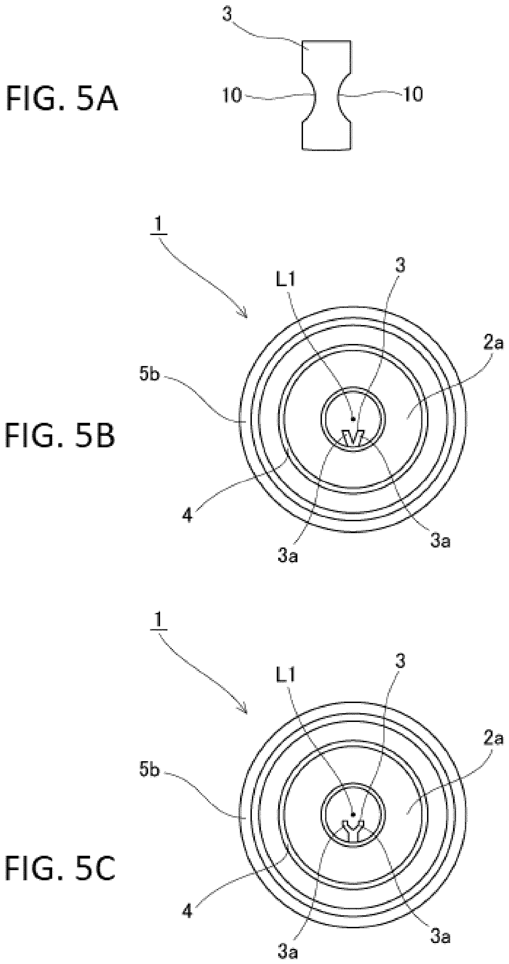

Fig. 5A is an enlarged view of another example of a support portion of the instrument for ear treatment according to the above embodiment of the present invention taken from an opening on a base end side in an axial direction. Fig. 5B is a diagram of another example of the instrument for ear treatment according to the above embodiment of the present invention taken from the opening on the base end side in the axial direction. Fig. 5C is a diagram of another example of the instrument for ear treatment according to the above embodiment of the present invention taken from the opening on the base end side in the axial direction.
Fig. 6 is a diagram of another example of the instrument for ear treatment according to the above embodiment of the present invention taken from the opening on the base end side in the axial direction.
Fig. 7 is a partial bottom view illustrating another example of the instrument for ear treatment according to the above embodiment of the present invention.

### Description of Embodiments

Embodiments of the instrument for ear treatment of the present invention are described below with reference to the drawings. It should be noted that the following description merely exemplifies preferred modes to understand the present invention and is not intended to limit the details of the present invention.

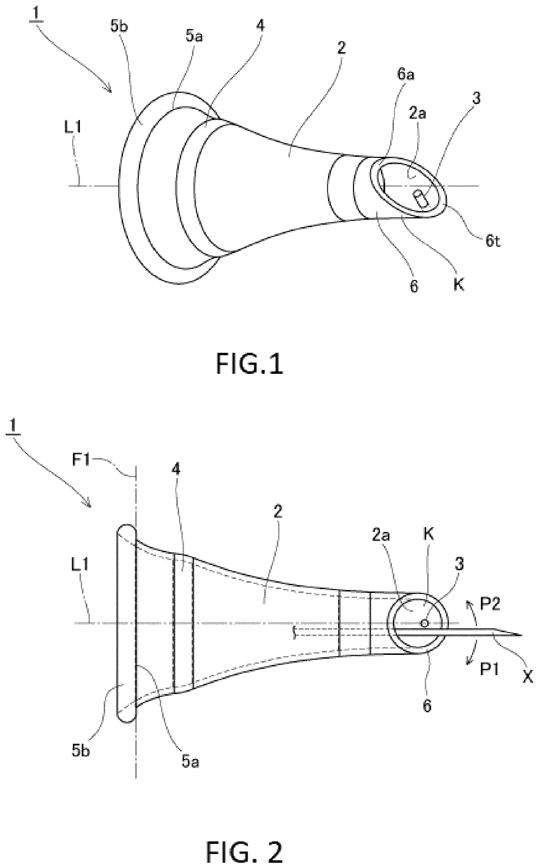

As illustrated in Figs. 1 and 2, an instrument 1 for ear treatment in the first embodiment includes an insertion portion 2 formed in a horn tubular shape to insert a tool X, such as a scalpel and an injection needle, while being inserted into the auditory canal (not shown, same below), and a support portion 3 to support the tool X inserted into the insertion portion 2.

The insertion portion 2 is formed in a tubular shape and in a horn shape slightly curved and flaring toward a base end. The insertion portion 2 has a distal end opened with a small diameter, and in contrast, the base end opened with a large diameter. In detail, the insertion portion 2 has a diameter gradually increasing at an approximately fixed rate from the distal end, and from a position approximately at the center in a direction of an axis L1, a diameter increasing to flare toward the base end greater than the distal end side and also to curve so as to slightly bend backward relative to the axis. It should be noted that the shape of the insertion portion 2 herein includes a substantially beheaded conical tube shape.

The insertion portion 2 has a diameter increasing from the approximate center in the direction of the axis L1 and then further increasing across a raised streak 4 circumferentially expanding about the axis L1. That is, the base end side from the center of the insertion portion 2 is widened in two steps.

The raised streak 4 close to the base end is formed by thickening part of the insertion portion 2.

The insertion portion 2 has an open end 5a on the base end side formed with a flange 5b jutting out the direction of the axis L1. The flange 5b expands to form an arc outline in a front view.

The insertion portion 2 in the area other than the flange 5b and the raised streak 4 has a thickness not particularly limited as long as the rigidity is secured and is formed to a thickness from 0.3 mm to 1.0 mm. The insertion portion 2 in the area other than the flange 5b and the raised streak 4 preferably has a thickness of 0.5 mm or more and 0.8 mm or less. The flange 5b and the raised streak 4 are thicker than the area other than them.

The flange 5b is formed on, but not particularly limited to, a surface F1 substantially vertical to the axis L1.

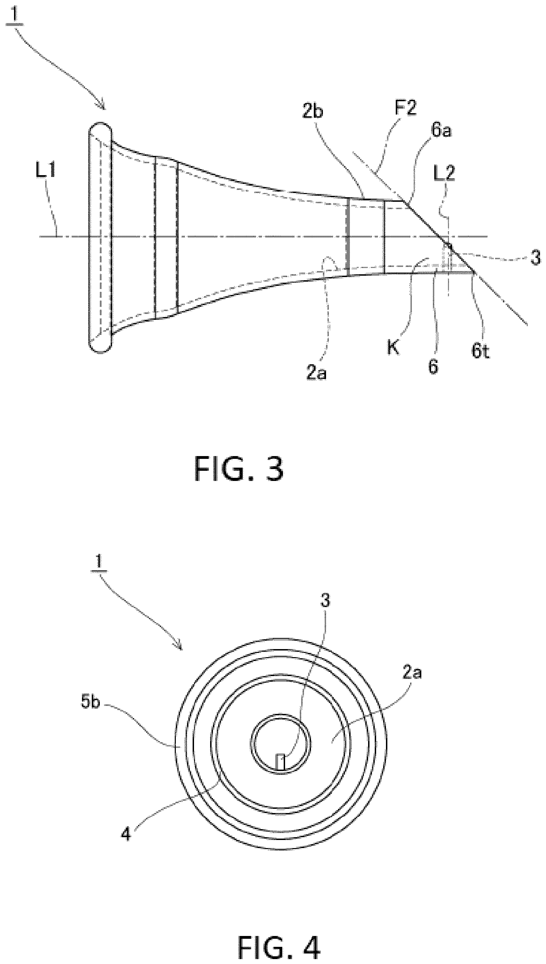

As illustrated in Fig. 3, the insertion portion 2 has a distal end portion 6 in a shape of obliquely cutting a roughly cylindrical surrounding wall portion to form a distal end surface 6a on a surface F2 inclined to the axis L1. The distal end surface 6a has an inclination angle of, but not particularly limited to, approximately 45 degrees in the example of Fig. 3. The inclination angle is preferably selected from, but does not have to be particularly limited to, angles between 30° and 90°. In a case of 90°, an opening on the distal end side is in a shape with no inclination.

The distal end portion 6 inclined toward a longest portion 6t extending longest in the direction of the axis L1 in the distal end portion 6 configures an inclined surrounding wall portion K. It should be noted that the angle made by the distal end surface 6a and an outer circumferential surface 2b of the insertion portion 2 may be formed smoothly not to be pointed.

The inclined surrounding wall portion K is formed to allow the opening to appear in a circular or oval shape in a front view and to have a distal edge in an arc shape. The inclined surrounding wall portion K is formed in a sharp shape in which the distal end surface 6a is inclined toward the longest portion 6t in a bottom view.

The support portion 3 is provided on an inner circumferential surface 2a of the insertion portion 2 and on the base end side of the longest portion 6t.

In the present embodiment, the support portion 3 is a projection projecting from the inner circumferential surface 2a. The support portion 3 has a substantially columnar solid shape with a diameter of approximately 1 mm.

The support portion 3 is directed in a direction of a substantially vertical intersection of an axis L2 of the support portion 3 and the axis L1 of the insertion portion 2. The support portion 3 is formed with a projection dimension of 1 mm or more and 5 mm or less. The projection dimension of the support portion 3 is preferably formed, in relation to the shape of the inclined surrounding wall portion K, with a dimension not to project from the surface F2 along the distal end surface 6a or to fall within less than 1 mm even when projecting from the surface F2.

The support portion 3 in the present embodiment is formed at a position of approximately 2.8 mm on the base end side from the longest portion 6t. However, the support portion 3 may be provided at a position of approximately 5 mm on the base end side from the longest portion 6t and is preferably formed at a position of 2.8 mm or more and 4 mm or less.

As described above, the distal end surface 6a in the present embodiment has an inclination angle relative to the axis L of approximately 45 degrees and the support portion 3 is formed at a position of 2.8 mm on the base end side from the longest portion 6t, thereby substantially avoiding projection of the support portion 3 from the opening.

The insertion portion 2 and the support portion 3 in the present embodiment are formed integrally as a single piece. The insertion portion 2 and the support portion 3 may preferably use a material of, but not particularly limited to, a synthetic resin, such as acryl, nylon, polyacetal (POM), polyphenylsulfone (PPSU), polyetheretherketone (PEEK), polyethylene terephthalate (PET), polycarbonate (PC), polylactic acid (PLA), polystyrene (PS), polyvinylidene fluoride (PVDF), polyamide-imide (PAI), and polyimide (PI), or a metal, such as stainless steel and titanium.

The support portion 3 does not have to be integrally formed with the insertion portion 2 as a single piece and may be attached to the inner circumferential surface 2a of the insertion portion 2. In this case, the insertion portion 2 and the support portion 3 may be formed from different materials.

As described above, the instrument 1 for ear treatment according to the present embodiment has the support portion 3 formed on the inner circumferential surface 2a of the insertion portion 2 and particularly on the inner circumferential surface 2a of the inclined surrounding wall portion K. Such a configuration allows, when the tool X such as a scalpel and an injection needle is inserted into the insertion portion 2, placement of a distal end area of the tool X on the support portion 3 to support the tool X as illustrated in Fig. 2. Particularly in the present embodiment, the support portion 3 is provided on the inner circumferential surface 2a of the inclined surrounding wall portion K, thereby allowing more stable support of the tool X while working by reducing the distance between the fulcrum to support the tool X with the support portion 3 and the distal end area of the tool X for surgery.

An effect of suppressing very slight vibration of the hand lower is thus exhibited for surgery to be practiced with caution in the small ear hole of approximately from 3 mm to 9 mm, such as a case of making a slit in an edge of the tympanic membrane and the like. Particularly, the instrument 1 for ear treatment facilitates suppression of vibration of the hand of approximately 0.3 mm, which sometimes occurs in not only a doctor without sufficient technique but even also a doctor with a high degree of technique and concentration.

In addition, the instrument 1 for ear treatment has the support portion 3 formed in a columnar shape and thus exhibits an effect of allowing a smooth change in the orientation of the distal end of the tool X to either direction of arrows P1 and P2 illustrated in Fig. 2 to facilitate surgery.

The instrument 1 for ear treatment also allows support of the tool X with the support portion 3 as a fulcrum and thus exhibits an effect of allowing suppression of slip, which sometimes occurs due to the smooth inner circumferential surface 2a when the tool X is inserted into the insertion portion 2 to be placed on the inner circumferential surface 2a.

As illustrated in Fig. 4, the instrument 1 for ear treatment in the present embodiment has the support portion 3 formed with a projection dimension of half or less of an inner diameter of the distal end portion 6 and with a diameter dimension of approximately one seventh of the inner diameter of the distal end portion 6. Accordingly, the instrument 1 for ear treatment allows satisfactory support of the tool X while maximally suppressing blocking the field of view in the insertion portion 2 of the support portion 3.

In addition, the instrument 1 for ear treatment according to the present embodiment is provided with the insertion portion 2 widened on the base end side from the center in two steps and with the raised streak 4 between the two-step flared widenings, and thus exhibits an effect of facilitating holding of the insertion portion 2.

The instrument 1 for ear treatment described in the above embodiment may be applied as follows.

That is, as illustrated in Fig. 5A, the support portion 3 formed in a columnar shape may be formed with a recess 10 in a penetration direction of the insertion portion 2 illustrated in Fig. 3 on the circumferential surface. The recess 10 may be formed in both sides of the support portion 3 across the axis L1 of the insertion portion 2 illustrated in Fig. 3, formed in one side of the surrounding wall, or formed in the entire circumference. The columnar shape of the support portion 3 does not have to project in the direction orthogonal to the axis L1 of the insertion portion 2 as long as the axis L2 of the support portion 3 illustrated in Fig. 3 projects upward to the axis L1 of the insertion portion 2.

The shape of the support portion 3 is not limited to the columnar shape and may be formed, as illustrated in Fig. 5B or 5C, in a V shape or a Y shape. In this case, the support portion 3 may be arranged to form the penetration direction between upper branches 3a and 3a of the V or Y shape or between the branch 3a and the inner circumferential surface 2a in parallel with the orientation of penetration in the direction of the axis L1. Such arrangement of the support portion 3 allows stable setting of the tool X between the branches 3a and 3a or between the branch 3a and the inner circumferential surface 2a to use the tool X.

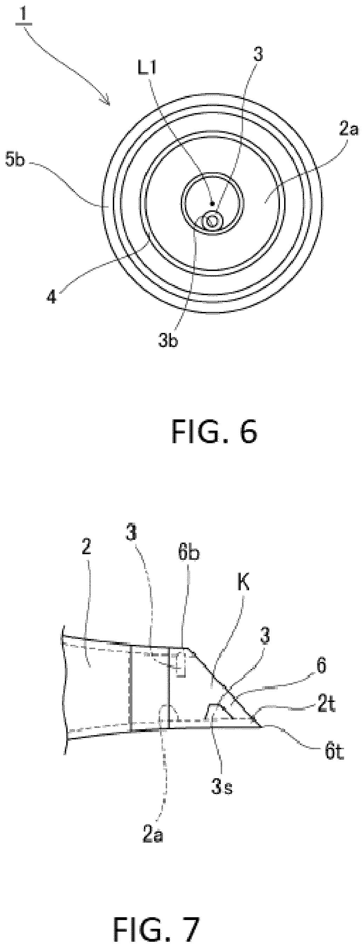

As still another example, the shape of the support portion 3 may be formed in a substantially O shape as illustrated in Fig. 6. In this case, a penetration direction of an opening 3b at the center of the support portion 3 may be arranged parallel to the orientation of penetration in the direction of the axis L1. Such arrangement of the support portion 3 allows stable setting of the tool X in the opening 3b at the center to use the tool X.

The support portion 3 may be formed in a shape other than the columnar shape, the O shape, the V shape, and the Y shape as long as a user of the instrument 1 for ear treatment is able to look into the ear and also to practice surgery while inserting the tool X for ear treatment, such as a scalpel and an injection needle, to support the tool with the support portion 3. For example, as illustrated in Fig. 7, the support portion 3 may be a flat small piece with a thickness of approximately 1 mm and provided with a flat plate surface 3s in a direction along the penetration direction of the insertion portion 2.

As illustrated by imaginary lines in Fig. 7, the support portion 3 in any of the various shapes described earlier may be formed on a surrounding wall facing the longest portion 6t of the inclined surrounding wall portion K, that is, the inner circumferential surface 2a on a shortest portion 6b side having the smallest projection of the inclined surrounding wall portion K in the direction of the axis L1. The support portion 3 provided at such a position allows surgery, in a case where an area other than the surgical site is swollen and the like, by setting the tool X to the support portion 3 while pressing the swelling area against the inclined surrounding wall portion K and maximally extend the surgical site.

In addition, a plurality of the support portion 3 in any of the various shapes described earlier may be formed on the inner circumferential surface 2a of the insertion portion 2 as long as a user, such as a doctor, is able to look into the ear and also to practice surgery by inserting the tool X.

The support portion 3 in any of the various shapes described earlier may have a surface that is formed not smoothly but as a rough surface capable of suppressing unintentional slip of the placed tool X.

The distal end portion 6 of the insertion portion 2 is not limited to the shape of obliquely cutting the cylinder relative to the axis L1 and may be in a shape having a surrounding wall portion flush with a surface orthogonal to the axis L1.

The support portion may be provided at an open end 2t on the distal end side of the insertion portion 2 illustrated in Fig. 7.

The insertion portion 2 may be formed from a transparent material. Formation of the insertion portion 2 from such a transparent material allows brightening inside the ear as much as possible and an indirect view of the state of the area pressed against the insertion portion 2.

Use of the instrument for ear treatment according to the present invention allows suppression of malpractice caused by, for example, movement of a subject during an operation or on the contrary shaking in operator's grip. The instrument for ear treatment according to the present invention is also expected to exhibit an effect of allowing, not only suppression of malpractice by facilitating surgery by a so-called skilled doctor, but also an easier operation for an operator without sufficient skills. In other words, use of the instrument for ear treatment according to the present invention allows operations that used to be practiced only by some skilled doctors in the past to be practiced by more doctors.

### Reference Signs List

- 1: Instrument for Ear Treatment
- 2: Insertion Portion
- 2a: Inner Circumferential Surface
- 3: Support Portion
- 4: Raised Streak
- 5a: Opening on Base End Side
- 5b: Flange
- 6: Distal End Portion
- 6a: Distal End Surface
- 6b: Shortest Portion
- 6t: Longest Portion
- F2: Inclined Surface
- K: Inclined Surrounding Wall Portion
- L: Axis
- X: Tool

## Claims

1. An instrument for ear treatment, comprising an insertion portion formed in a horn tubular shape and configured to be inserted into an external acoustic meatus, wherein
a support portion configured to support a tool to be inserted into the insertion portion is provided on an inner circumferential surface or at an open end on a distal end side of the insertion portion.

2. The instrument for ear treatment according to Claim 1, wherein the support portion is formed from a projection projecting from the inner circumferential surface of the insertion portion.

3. The instrument for ear treatment according to Claim 1 or 2, wherein the support portion is formed on a distal end side in a direction of an axis of the insertion portion.

4. The instrument for ear treatment according to any one of Claims 1 through 3, wherein
the insertion portion has a distal end portion as an inclined surrounding wall portion having a shape obtained by obliquely cutting a surrounding wall in a cylindrical shape relative to an axis of the surrounding wall, and
the inclined surrounding wall portion has an inner circumferential surface formed with the support portion.

5. The instrument for ear treatment according to any one of Claims 1 through 3, wherein
the insertion portion has a distal end portion as an inclined surrounding wall portion having a shape obtained by obliquely cutting a surrounding wall in a cylindrical shape relative to an axis of the surrounding wall, and
the support portion is formed on an inner circumferential surface on a shortest portion side of the inclined surrounding wall portion facing a longest portion of the distal end portion most projecting in a direction of an axis.

6. The instrument for ear treatment according to any one of Claims 1 through 5, wherein the inner circumferential surface is provided with a plurality of the support portions.

7. The instrument for ear treatment according to any one of Claims 1 through 6, wherein the support portion is formed in a columnar shape.

8. The instrument for ear treatment according to any one of Claims 1 through 6, wherein the support portion is formed in an O shape, a V shape, or a Y shape.

9. The instrument for ear treatment according to any one of Claims 1 through 8, wherein the support portion has a surface formed with a recess to support the tool.

10. The instrument for ear treatment according to any one of Claims 1 through 9, wherein the support portion has a height of 1 mm or more and 5 mm or less.

11. The instrument for ear treatment according to any one of Claims 1 through 10, wherein the insertion portion has an outer circumferential surface provided with a raised streak extending about the axis.

12. The instrument for ear treatment according to any one of Claims 1 through 11, wherein the instrument for ear treatment is formed from a transparent material.
